(19) Europäisches Patentamt
European Patent Office
Office européen des brevets

(11) **EP 0 569 618 B1**

(12) **EUROPÄISCHE PATENTSCHRIFT**

(45) Veröffentlichungstag und Bekanntmachung des Hinweises auf die Patenterteilung:
**02.01.1997 Patentblatt 1997/01**

(51) Int. Cl.⁶: **A61M 5/142**, F04B 49/10, F04B 17/04

(21) Anmeldenummer: **92118257.2**

(22) Anmeldetag: **26.10.1992**

(54) **Dosiergerät zur gesteuerten Abgabe einer Flüssigkeit**

Dosing device for the controlled release of a liquid

Dispositif de dosage de diffusion contrôlée d'un liquide

(84) Benannte Vertragsstaaten:
**DE FR GB IT NL SE**

(30) Priorität: **12.05.1992 EP 92107994**

(43) Veröffentlichungstag der Anmeldung:
**18.11.1993 Patentblatt 1993/46**

(73) Patentinhaber:
• **Siemens-Elema AB**
**171 95 Solna 1 (SE)**
Benannte Vertragsstaaten:
**SE**

• **SIEMENS AKTIENGESELLSCHAFT**
**80333 München (DE)**
Benannte Vertragsstaaten:
**DE FR GB IT NL**

(72) Erfinder: **Slettenmark, Bruno**
**S-175 60 Järfälla (SE)**

(56) Entgegenhaltungen:
**EP-A- 0 110 117**  **EP-A- 0 183 351**
**EP-A- 0 317 705**  **US-A- 4 360 019**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Dosiergerät zur gesteuerten Abgabe einer Flüssigkeit aus einem Vorratsbehälter mit einer Kolbenpumpe, deren Kolben durch eine ein- und ausschaltbare elektromagnetische Betätigungseinrichtung aus einer Ruhelage gegen einen Anschlag eines den Kolben umgebenden Zylindergehäuses bewegbar ist, mit einem Pumpratengeber, der für jede einzelne Pumpaktion ein Einschaltsignal für die Betätigungseinrichtung erzeugt, und mit einer Detektoreinrichtung, die beim Auftreffen des Kolbens gegen den Anschlag ein Ausgangssignal erzeugt, das in einer Auswerteeinrichtung zur Erzeugung eines Ausschaltsignals für die Betätigungseinrichtung herangezogen wird.

Ein derartiges, aus der EP-A-0 317 705 bekanntes Dosiergerät dient zum Injizieren von Insulin aus einem Vorratsbehälter in den Körper eines Patienten. Das als batteriebetriebenes Implantat ausgebildete bekannte Gerät enthält zum Dosieren und Fördern des Insulins eine Kolbenpumpe, deren Kolben auf elektromagnetischem Wege gegen einen Anschlag beschleunigt wird. Um die Pumpfunktion in ihrem Verlauf überwachen zu können, ist eine Detektoreinrichtung vorgesehen, die den Bewegungsverlauf des Kolbens indirekt erfaßt und das Auftreffen des Kolbens gegen den Anschlag detektiert. Das dabei von der Detektoreinrichtung erzeugte Ausgangssignal wird zum Abschalten der elektromagnetischen Erregung herangezogen, wodurch sichergestellt wird, daß die Pumpe am Ende jeder Pumpaktion abgeschaltet ist und so keine weitere Energie der Batterie entzogen wird.

Der Erfindung liegt die Aufgabe zugrunde, den Energieverbrauch der Kolbenpumpe noch weiter zu vermindern um die Lebensdauer des Geräts zu verlängern und/oder seine Größe durch Anwendung einer kleineren Batterie zu verkleinern und darüberhinaus die Beeinträchtigung der zu pumpenden Flüssigkeit, der Pumpe selbst und der Elektronik durch die Kolbenbewegung zu verringern, sowie das Geräusch zu reduzieren und noch die Möglichkeit den Zustand des Gerätes zu diagnostizieren

Gemäß der Erfindung wird die Aufgabe dadurch gelöst, daß bei dem Dosiergerät der eingangs angegebenen Art die Auswerteeinrichtung das Ausschaltsignal nach Ablauf eines auf das Auftreten des Einschaltsignals folgenden Einschaltzeitintervalls erzeugt, dessen Länge kürzer bemessen ist, als die bei störungsfreiem Betrieb des Dosiergeräts für die Bewegung des Kolbens aus der Ruhelage gegen den Anschlag benötigte Zeit, und daß das Einschaltzeitintervall verlängert wird, wenn bei einer vorgegebenen Anzahl zurückliegender Pumpaktionen die Zahl der detektierten Anschläge des Kolbens einen vorgegebenen Wert unterschreitet.

Dabei ist im einfachsten Fall vorgesehen, daß das Einschaltzeitintervall ausreichend lang bemessen ist, um den Kolben bei störungsfreiem Betrieb des Dosiergeräts gegen den Anschlag zu beschleunigen, und daß die Auswerteeinrichtung zur Verlängerung des Einschaltzeitintervalls das Ausgangssignal der Detektoreinrichtung als Ausschaltsignal heranzieht. Alternativ hierzu wird das Einschaltzeitintervall bei jedem Ausbleiben einer Detektion des Auftreffens des Kolbens gegen den Anschlag um einen vorgegebenen Betrag verlängert, so daß automatisch sehr schnell ein Wert für das Einschaltzeitintervall gefunden wird, der zur Beschleunigung des Kolbens gegen den Anschlag ausreicht. Das Einschaltzeitintervall kann periodisch, automatisch oder manuell von dem Patienten selbst oder vom Arzt beim Arztbesuch auf den ursprünglichen Wert zurückgestellt werden, da die Abweichungen von zeitweiliger Natur sein können. In dem ersten Fall wird die elektromagnetische Betätigungseinrichtung ausgeschaltet, bevor der beschleunigte Kolben auf den Anschlag trifft. Durch die kinetische Energie des Kolbens und der Flüssigkeit sowohl vor als auch hinter dem Kolben sowie die Energie des in der elektromagnetischen Betätigungseinrichtung aufgebauten magnetischen Feldes wird der Kolben auch nach dem Ausschalten der elektromagnetischen Erregung weiter bis an den Anschlag getrieben. Auf diese Weise wird der Energieverbrauch zum Antreiben des Kolbens erheblich herabgesetzt.

Ein weiterer Vorteil des erfindungsgemäßen Dosiergeräts besteht darin, daß durch das vorzeitige Abschalten der elektromagnetischen Betätigungseinrichtung der Kolben mit geringerer Geschwindigkeit als bei dem bekannten Dosiergerät gegen den Anschlag trifft und daß auch die maximale Geschwindigkeit, die der Kolben erreicht, geringer ist, als bei dem bekannten Gerät. Dadurch wird die mechanische Beanspruchung der zu fördernden Flüssigkeit verringert, was insbesondere bei Insulin und anderen flüssigen Medikamenten von Bedeutung ist, weil u.a. die in ihnen enthaltenen Proteine sehr empfindlich in bezug auf mechanische Beanspruchungen sind. Durch die verminderte Auftreffgeschwindigkeit des Kolbens gegen den Anschlag wird die Flüssigkeit in geringerem Maße als bisher zwischen den Auftreffflächen des Kolbens und des Anschlags zusammengepreßt. Ferner werden auch die mechanische Beanspruchung der Kolbenpumpe, Vibrationen in elektronischen Schaltungen und deren Geräuschentwicklung, die dem Patient lästig sein können, reduziert. Durch die verminderte Maximalgeschwindigkeit des Kolbens werden Turbulenzen in der Flüssigkeit vermieden und Scherkräfte innerhalb der Flüssigkeit verringert. Durch Beibehaltung der Beschleunigung des Kolbens unter einem gewissen, kritischen Wert, wird außerdem verhindert, daß hinter dem Kolben, also auf dessen Saugseite, der Druck in der Flüssigkeit unter ihren Dampfdruck sinkt und dadurch Kavitationserscheinungen auftreten, welche eine hohe mechanische Beanspruchung der Flüssigkeit darstellen und außerdem zu Beschädigungen, zum Beispiel Erosion, der Kolbenpumpe sowie aufgrund der dabei auftretenden mechanischen Druckwellen zu Fehldetektionen durch die Detektoreinrichtung führen können. Unter anderem sind Insulinlösungen gegen hydrophobe Wechselwirkung, d.h. in der Grenzfläche zwischen Flüssigkeits- und Gasphase sehr empfindlich und auch aus diesem Grund ist die Verhinderung von Kavitation wichtig.

Obwohl die erfindungsgemäße Kolbenpumpe mit einer minimalen Energie betrieben wird, ist stets ein sicherer Betrieb der Kolbenpumpe gewährleistet, weil im Falle des Ausbleibens von Detektionen des Auftreffens des Kolbens gegen den Anschlag das Einschaltzeitintervall für die elektromagnetische Erregung verlängert wird. Die Verlängerung kann automatisch nach Ablauf einer vorgegebenen Zeitspanne oder manuell durch eine Bedienperson rückgängig gemacht werden.

Entsprechend einer vorteilhaften Weiterbildung des erfindungsgemäßen Dosiergeräts ist vorgesehen, daß die Auswerteeinrichtung zur Ermittlung des für eine vollständige Pumpaktion kürzest möglichen Einschaltzeitintervalls das Einschaltzeitintervall ausgehend von einem Wert, bei dem ein Ausgangssignal der Detektoreinrichtung erhalten wird, in aufeinanderfolgenden Pumpaktionen schrittweise verkürzt, bis das Ausgangssignal der Detektoreinrichtung ausbleibt, und daß anschließend das Einschaltzeitintervall um einen vorgegebenen Betrag verlängert wird, wobei dieser Vorgang mit gleichmäßigen Intervallen wiederholt wird um das Einschaltzeitintervall dynamisch an veränderten Energiebedarf der Pumpe anzupassen. Hierdurch wird das Einschaltzeitintervall, während dessen die elektromagnetische Betätigungseinrichtung eingeschaltet ist, automatisch an veränderte Betriebsbedingungen der Kolbenpumpe angepaßt. Hierzu gehören beispielsweise Änderungen der Pumprate, Änderungen der Reibung zwischen Kolben und Zylindergehäuse, Verschleiß und Veränderungen von beweglichen Teilen Behinderungen der Flüssigkeitsströmung vor oder hinter dem Kolben beispielsweise durch Verstopfung, Luft- und Gasvorkommen in der Flüssigkeit oder sonstige Veränderungen der Flüssigkeit. Die Ermittlung des für eine effektive Pumpaktion kürzest möglichen Einschaltzeitintervalls kann ständig oder in bestimmten Zeitabständen wiederholt werden.

Um sicherzustellen, daß bei der Ermittlung des für eine effektive Pumpaktion kürzesten Zeitintervalls auch zu dem Zeitpunkt, in dem das Ausgangssignal der Detektoreinrich-tung ausbleibt, noch eine vollständige Pumpaktion vorliegt, ist in vorteilhafter Weise vorgesehen, daß die Detektoreinrichtung das Auftreffen des Kolbens gegen den Anschlag mit einer vorgegebenen Empfindlichkeitsschwelle detektiert und daß die Schrittweite, mit der die Verkürzung des Einschaltzeitintervalls erfolgt, auf einen Wert bemessen ist, der kleiner ist, als die Differenz zwischen dem Einschaltzeitintervall, bei dem der Kolben gerade noch den Anschlag erreicht und dem Zeitintervall, bei dem das Auftreffen des Kolbens gegen den Anschlag durch Überschreiten der Empfindlichkeitsschwelle detektiert wird.

Zur Überwachung der Funktionsfähigkeit der Kolbenpumpe ist entsprechend einer Weiterbildung des erfindungsgemäßen Dosiergeräts vorgesehen, daß an der Auswerteeinrichtung ein Fehlermelder angeschlossen ist, der ein Fehlermeldungssignal abgibt, wenn die eingestellte Länge des Einschaltzeitintervalls einen vorgegebenen Maximalwert überschreitet oder einen vorgegebenen Mindestwert unterschreitet.

Gemäß einer vorteilhaften Weiterbildung des erfindungsgemäßen Dosiergeräts ist an der Auswerteeinrichtung ein Datenspeicher angeschlossen, in den der aktuelle Wert für das Einschaltzeitintervall fortlaufend oder beim Ausbleiben eines Ausgangssignals der Detektoreinrichtung eingelesen wird. Dabei wird zusätzlich in vorteilhafter Weise zusammen mit dem Wert für das Einschaltzeitintervall auch der zugehörige aktuelle Wert der Pumprate und die Zeitpunkte des Beginns der Bewegung und des Anschlags des Kolbens abgespeichert. Wegen der Induktanz in dem elektrischen Kreis erreicht der Strom erst nach einer gewissen Zeit so eine Stärke, daß die statischen Kräfte überwindet werden und die Beschleunigung des Kolbens anfängt. Das vereinzelte Ausbleiben eines Ausgangssignals der Detektoreinrichtung bei einzelnen Pumpaktionen muß nämlich nicht notwendigerweise schon eine Störung der Funktion der Kolbenpumpe bedeuten. Erst wenn sich die Fälle, bei denen eine Detektion des Auftreffens des Kolbens gegen den Anschlag ausbleibt, häufen, führt dies zu einer Fehlermeldung. In einem solchen Fall läßt sich die Störungsursache im Nachhinein durch Auswertung der in dem Datenspeicher chronologisch abgelegten Ereignisse, in denen ein Auftreffen des Kolbens gegen den Anschlag nicht detektiert wurde, ermitteln. Dabei kann die Ermittlung der Störungsursache entweder automatisch in der Auswerteeinrichtung oder durch eine Bedienperson erfolgen, die die Daten aus dem Datenspeicher ausliest. Auf entsprechende Weise läßt sich auch eine künftige mögliche Störung des Pumpbetriebs im voraus feststellen.

Zur Erläuterung der Erfindung wird im folgenden auf die Figuren der Zeichnung bezug genommen. Im einzelnen zeigen

Figur 1 einen Querschnitt durch den mechanischen Teil einer Kolbenpumpe,

Figur 2 das Prinzipschaltbild einer Steuer- und Überwachungsschaltung für die in Figur 1 gezeigte Kolbenpumpe,

Figur 3 Kurvenverläufe des Erregerstroms für die Kolbenpumpe, der Geschwindigkeit des Kolbens und des Ausgangssignals einer Detektoreinrichtung der in Figur 1 gezeigten Kolbenpumpe und

Figur 4 den prinzipiellen Verlauf der Veränderung des Einschaltzeitintervalls bei der Ermittlung des für eine vollständige Pumpaktion kürzest erforderlichen Wertes für das Einschaltzeitintervall.

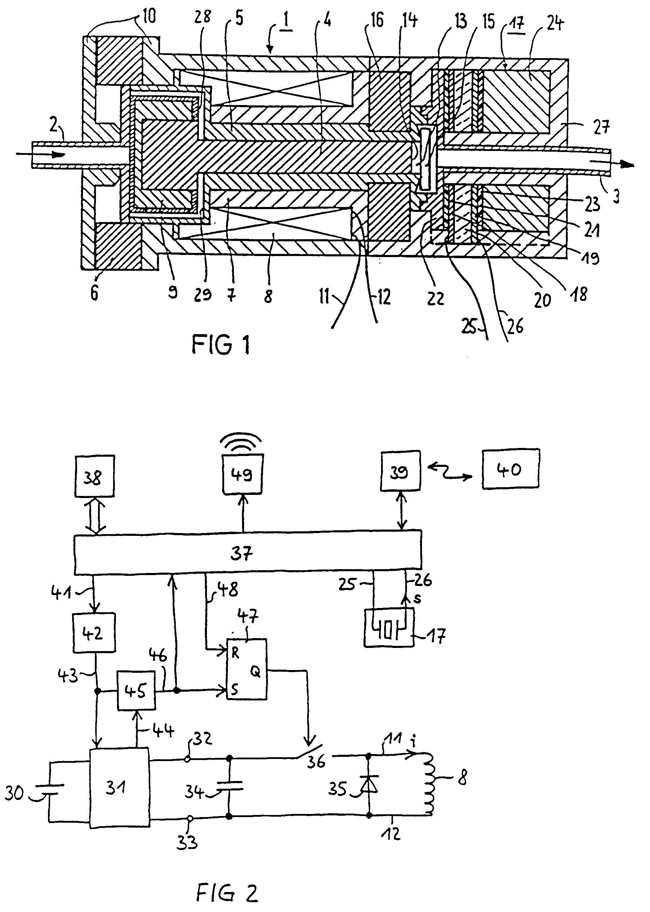

Das in Figur 1 im Querschnitt dargestellte Ausführungsbeispiel für eine Kolbenpumpe 1 eines Dosiergeräts weist einen Einlaßkanal 2 und einen Auslaßkanal 3 für eine zu fördernde Flüssigkeit auf. Die Kolbenpumpe 1 kann beispielsweise Bestandteil eines implantierbaren Medikamentendosiergeräts sein und ist in diesem Fall an ihrem Einlaßkanal 2 mit einem hier nicht gezeigten Vorratsbehälter für das Medikament, beispielsweise Insulin, verbunden und an dem Auslaßkanal 3 an einer hier ebenfalls nicht gezeigten Katheteranordnung zum Applizieren des Medikaments in einem Organismus angeschlossen. Die Kolbenpumpe 1 weist einen Kolben 4 auf, der in einem Zylindergehäuse 5 untergebracht ist. Der Antrieb des Kolbens 4 erfolgt durch eine elektromagnetische Betätigungseinrichtung bestehend aus einem im Bereich des Einlaßkanals 2 angeordneten Permanentmagnet 6, einem auf dem Zylindergehäuse 5 sitzenden Stator 7 mit einer Statorwicklung 8 und aus einem mit dem Kolben 4 fest verbundenen gekapselten Anker 9, der im wesentlichen zwischen dem Permanentmagnet 6 und dem Stator 7 angeordnet ist. Die Polschuhe des Permanentmagneten 6 und des Stators 7 sind jeweils mit 10 bezeichnet. Die Statorwicklung 8 ist über Anschlußleitungen 11 und 12 mit einer in Figur 2 näher gezeigten Steuerschaltung verbunden. Der Hubraum 13 des Kolbens 4 ist von der dem Auslaßkanal 3 zugewandten Stirnfläche 14 des Kolbens 4, dem Zylindergehäuse 5 und einem Ventil 15 begrenzt, das durch die Kraft eines Magneten 16 in einer den Hubraum 13 abdichtenden Stellung gehalten wird.

Der Kolben 4 wird durch den Penmanentmagneten 6 in seiner in Figur 1 gezeigten Ruhelage gehalten. Bei Erregung der Statorwicklung 8 wird der Kolben 4 aus seiner Ruhelage heraus in Richtung auf das Ventil 15 beschleunigt und preßt dabei die sich im Kolbenhubraum 13 befindende Flüssigkeit durch das sich gegen die Rückstellkraft des Magneten 16 öffnende Ventil 15 in den Auslaßkanal 3. Zugleich wird auf der Rückseite des Kolbens 4 durch den Einlaßkanal 2 hindurch Flüssigkeit aus dem Vorratsbehälter angesaugt, die beim Abschalten der Erregung der Statorwicklung 8, wenn der Kolben 4 durch die Kraft des Permanentmagneten 6 in seine Ruhelage zurückbewegt wird, durch den Spalt zwischen dem Kolben 4 und dem ihn umgebenden Zylindergehäuse 5 hindurch in den Hubraum 13 gesaugt wird

Im Bereich des Auslaßkanals 3 ist konzentrisch zu diesem eine Detektoreinrichtung in Form eines Geräuschsensors 17 angeordnet. Dieser besteht aus einer beidseitig mit Metallschichten 18 und 19 versehenen piezokeramischen Ringscheibe 20, die an einer Seite über eine Isolierschicht 21 an einem Flansch 22 der Kolbenpumpe 1 befestigt ist und an der gegenüberliegenden Seite über eine weitere Isolierschicht 23 mit einer Koppelmasse 24 verbunden ist. Die Metallschichten 18 und 19 sind mit Anschlußleitungen 25 und 26 versehen, die nach außen geführt sind. Der Geräuschsensor 17 ist in einem Gehäuse 27 angeordnet, das an der Kolbenpumpe 1 fixiert ist.

Der mit dem Kolben fest verbundene Anker 9 weist eine in Richtung auf den Auslaßkanal 3 weisende Ringfläche 28 auf, der eine als Anschlag 29 ausgebildete Fläche des Zylindergehäuses 5 gegenüber liegt. Bei jeder vollständigen Pumpaktion trifft der Kolben 4 mit seinem Anker 9 gegen den Anschlag 29, wobei ein sich aus dem allgemeinen Pumpgeräusch heraushebendes Anschlaggeräusch erzeugt wird. Dieses Anschlaggeräusch wird über das Zylindergehäuse 5 und den Flansch 22 auf den Geräuschsensor 17 übertragen und in ein elektrisches Ausgangssignal umgesetzt, das sich an den Anschlußleitungen 25 und 26 abnehmen läßt.

In Figur 2 ist das Prinzipschaltbild einer Steuer- und Überwachungsschaltung für die in Figur gezeigte Kolbenpumpe 1 dargestellt. An einer Batterie 30 ist eine Spannungsvervielfacherschaltung 31 angeschlossen, an deren Ausgangsanschlüssen 32 und 33 ein Ladekondensator 34 liegt. Die Statorwicklung 8 der Kolbenpumpe 1 ist zusammen mit einer zwischen ihren Anschlußleitungen 11 und 12 liegenden Freilaufdiode 35 über einen steuerbaren Schalter 36 parallel an den Ladekondensator 34 geschaltet. Die Steuerung der Erregung der Statorwicklung 8 erfolgt mittels einer übergeordneten Steuereinheit 37, die mit einem Datenspeicher 38 verbunden ist und an der eine Telemetrieeinrichtung 39 zur Kommunikation mit einem äußeren Programmiergerät 40 angeschlossen ist. Mittels des Programmiergeräts 40 werden unter anderem auch Werte für die Förderrate der zu pumpenden Flüssigkeit in die Steuereinheit 37 übertragen. Dabei lassen sich eine Vielzahl unterschiedlicher Förderraten in dem Speicher 38 abspeichern, die automatisch nach einem vorgegebenen Programmablauf oder durch Betätigung des Programmiergeräts 40 aufgerufen und über eine Steuerleitung 41 in einen Pumpratengeber 42 übertragen werden. Der Pumpratengeber 42 erzeugt an seiner Ausgangsleitung 43 eine Folge von einzelnen Impulsen, wobei jeder Einzelimpuls eine einzelne Pumpaktion der Kolbenpumpe 1 definiert und die Impulsfolgefrequenz proportional zu der aktuellen Förderrate ist. Die Ausgangsleitung 43 des Pumpratengebers 42 ist mit einem Aktivierungseingang der Spannungsvervielfacherschaltung 31 verbunden. Diese wird bei jedem Einzelimpuls eingeschaltet und erzeugt an einem Kontrollausgang 44 ein Signal, sobald der Ladekondensator 34 auf eine vorgegebene Mindestspannung aufgeladen ist. Beim Auftreten des Signals an dem Kontrollausgang 44 und dem vorherigen Auftreten eines Einzelimpulses auf der Steuerleitung 43 wird von einer Logikschaltung 45 auf einer Ausgangssignalleitung 46 ein Ausgangssignal erzeugt, das sowohl der Steuereinheit 37 als auch dem Setzeingang S einer bistabilen Kippschaltung 47 zugeführt wird. Dabei erzeugt die bistabile Kippschaltung 47 an ihrem Ausgang Q ein Signal, das dem steuerbaren Schalter 36 als Einschaltsignal zuführbar ist. Gleichzeitig wird in der Steuereinheit 37 ein Zeitintervall gestartet, nach dessen Ablauf die bistabile Kippstufe 47 an ihrem Rücksetzeingang R über eine Steuerleitung 48 zurückgesetzt wird und an ihrem Ausgang Q ein Ausschaltsignal für den steuerbaren Schalter 36 erzeugt. An der Steuereinheit 37 ist ferner der Geräuschsensor 17 mit seinen beiden Anschlußleitungen 25 und 26 angeschlossen. Das Ausgangssignal des Geräuschsensors 17 wird in einer hier nicht eigens gezeigten Auswerteeinrichtung, die ein integraler Bestandteil der Steuereinheit 37 ist, analysiert. Schließlich ist an der Steuereinheit 37 ein Fehlermelder 49 angeschlossen, bei dem im vorliegenden Fall eine Fehlermeldung durch Abgabe eines akustischen Signals erfolgt; es ist aber auch möglich, daß die Fehlermeldung durch Abgabe eines elektrischen Reizimpulses an den Patienten oder durch telemetrische Überwachung des Fehlermeldesignals an das Programmiergerät 40 erfolgt.

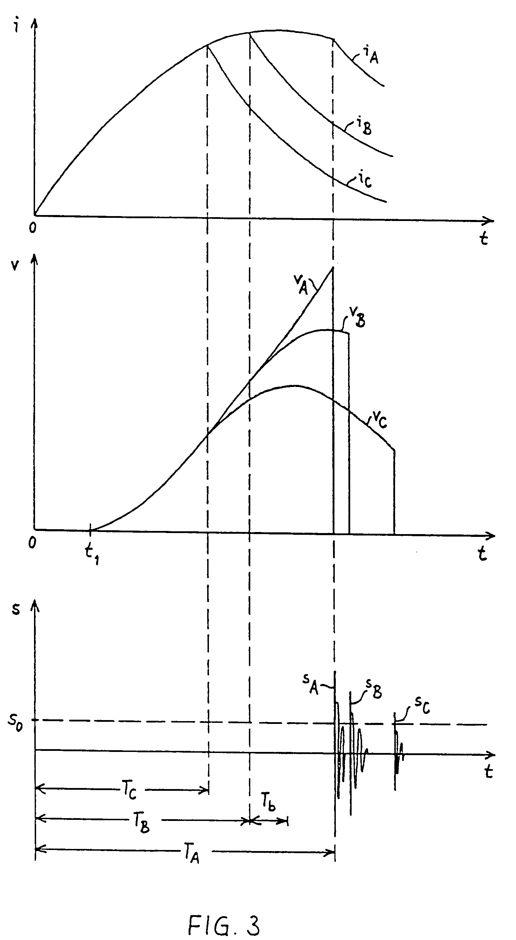

Figur 3 zeigt in drei Diagrammen von oben nach unten den Strom i durch die Statorwicklung 8, die Geschwindigkeit v des Kolbens 4 und das Ausgangssignal s des Geräuschsensors 17 für jeweils drei unterschiedliche Einschaltzeitintervalle $T_A$, $T_B$ und $T_C$, während derer der steuerbare Schalter 36 geschlossen ist. Die den unterschiedlichen Zeitintervallen $T_A$, $T_B$ und $T_C$ zugeordneten Kurvenverläufe des Stromes i, der Kolbengeschwindigkeit v und des Ausgangssignals s sind entsprechend mit A, B, C indiziert.

Zum Zeitpunkt t=0 wird eine Pumpaktion gestartet, indem der Schalter 36 durch ein Einschaltsignal am Ausgang Q der Kippstufe 47 geschlossen wird. Der Ladekondensator 34 entlädt sich über die Statorwicklung 8, in der ein Strom i in Form einer gedämpften Sinusschwingung fließt. Wenn das durch den Strom i in der Statorwicklung 8 erzeugte Statormagnetfeld die Rückhaltekraft des Permanentmagneten 6, die Haftreibung des Kolbens 4 im Zylindergehäuse 5 und die Ventilkraft übersteigt, der Zeitpunkt $t=t_1$, in Figur 3, wird der Kolben 4 in Richtung auf das Ventil 15 beschleunigt, wobei die Kolbengeschwindigkeit v progressiv zunimmt.

Im Fall A, der der aus der eingangs genannten EP-A-0 317 705 bekannten Pumpensteuerung entspricht, bleibt der Schalter 36 so lange geschlossen, bis der Kolben 4 gegen den Anschlag 29 trifft. Das dabei entstehende typische Ausgangssignal $s_A$ des Geräuschsensors 17 übersteigt dessen Empfindlichkeitsschwelle $s_0$ zur Detektion des Auftreffens des Kolbens 4 gegen den Anschlag 29 und wird zur Auslösung eines das Einschaltzeitintervall $T_A$ beendenden Ausschaltsignals für den Schalter 36 herangezogen. Wie Figur 3 zeigt, erreicht die Kolbengeschwindigkeit $v_A$ am Ende des Einschaltzeitintervalls $T_A$ ihren maximal möglichen Wert, so daß der Kolben 4 mit der höchstmöglichen Anschlagenergie gegen den Anschlag 29 trifft. Im Dauerbetrieb der Kolbenpumpe 1 kann diese hohe Anschlagenergie zu mechanischen Beschädigungen der Kolbenpumpe 1 und zu einer unzulässig hohen mechanischen Beanspruchung der zu för-dernden Flüssigkeit führen. Außerdem ist, wie aus dem Stromverlauf $i_A$ ersichtlich, der Energieverbrauch vergleichsweise hoch. Darüberhinaus kann das Anschlagsgeräusch dem Patienten und seiner Umgebung lästig sein.

Entsprechend einer ersten Ausführungsmöglichkeit der Erfindung ist für das Einschaltzeitintervall ein fester Wert $T_B$ vorgesehen, der beispielsweise aufgrund von Versuchen ermittelt wird und einerseits so kurz bemessen ist, daß die Erregung der Statorwicklung 8 abgeschaltet wird, bevor der Kolben 4 gegen den Anschlag 29 trifft, andererseits aber ausreichend lang bemessen ist, um bei störungsfreiem Betrieb der Kolbenpumpe 1 eine ausreichend hohe Anschlagenergie des Kolbens 4 zu erhalten, so daß das Ausgangssignal $s_B$ des Geräuschsensors 17 die Empfindlichkeitsschwelle $s_0$ sicher übersteigt. Zu Beginn der Pumpaktion wird also durch Setzen der Kippstufe 47 ein Einschaltsignal für den Schalter 36 erzeugt. Nach Ablauf des vorbestimmten Einschaltzeitintervalls $T_B$ wird die Kippstufe 47 zurückgesetzt und der Schalter 36 durch ein Ausschaltsignal am Ausgang Q der Kippstufe 47 geöffnet. Die zum Abschaltzeitpunkt in der Statorwicklung 8 gespeicherte magnetische Energie bewirkt, daß der Strom $i_B$ weiter durch die Statorwicklung 8 und die Freilaufdiode 35 fließt, wobei der Strom $i_B$ aufgrund des Innenwiderstands der Statorwicklung 8 exponentiell abklingt.

Durch die kinetische Energie des Kolbens 4 und der Flüssigkeit sowohl vor als auch hinter dem Kolben 4 sowie die zum Abschaltzeitpunkt in der Statorwicklung 8 gespeicherte magnetische Energie wird der Kolben 4 weiter bis an den Anschlag 29 getrieben und erzeugt in dem Geräuschsensor 17 das Ausgangssignal $s_B$. Wie der Figur 3 zu entnehmen ist, ist sowohl der Stromverbrauch als auch die maximale Kolbengeschwindigkeit und die Anschlagenergie des Kolbens 4 geringer, als bei dem oben beschriebenen bekannten Ansteuerungsmodus A. Es soll notiert werden, daß die Anschlagsenergie eine quadratische Funktion der Anschlagsgeschwindigkeit ist. Auch die Kolbenbeschleunigung wird geringer als bei dem früheren Ansteuerungsmodus, was vorteilhaft ist, um Kavitation zu verhindern. Dadurch ist die mechanische Beanspruchung sowohl der Kolbenpumpe 1 als auch der zu pumpenden Flüssigkeit geringer.

Wenn aufgrund von Störungen, gleich welcher Art, bei einer vorgegebenen Anzahl zurückliegender Pumpaktionen die Zahl der von dem Geräuschsensor 17 detektierten Anschläge des Kolbens 4 einen vorgegebenen Wert unterschreitet, wird dies von der Steuereinheit 37 registriert. Die Steuereinheit 37 verlängert daraufhin das Einschaltzeitintervall, nach dessen Ablauf die bistabile Kippstufe 47 bei den folgenden Pumpaktionen jeweils zurückgesetzt wird. Dies geschieht entweder dadurch, daß das Zeitintervall $T_B$ um einen vorgegebenen Betrag $T_b$ verlängert wird, oder daß das Ausschaltsignal für den Schalter 36 erst beim Auftreten eines das Auftreffen des Kolbens 4 gegen den Anschlag 29 detektierenden Ausgangssignals s des Geräuschsensors 17 erzeugt wird. Gleichzeitig wird das Störereignis zusammen mit anderen Pumpparametern wie z.B. der aktuellen Pumprate, dem Zeitintervall $T_A$ und dem Zeitpunkt $t_1$, in dem Speicher 38 abgespeichert, so daß es zur Fehlerauswertung von einem Arzt mittels des Programmiergeräts 40 abgerufen werden kann. Außerdem wird das Störereignis dem Patienten durch den Fehlermelder 49 akustisch oder beispielsweise durch einen Reizstromimpuls mitgeteilt. Alternativ bekommt er eine Fehlermeldung erst bei dem nächsten Kontakt mit der Telemetrieeinrichtung oder bei dem nächsten Arztbesuch. Das verlängerte Zeitintervall kann über das Programmiergerät 40 manuell auf den früheren Wert $T_B$ zurückgestellt werden. Es ist aber auch möglich, daß die Zurückstellung auf den früheren Wert $T_B$ automatisch durch Ablauf einer programmierten Zeit in der Steuereinheit 37 erfolgt.

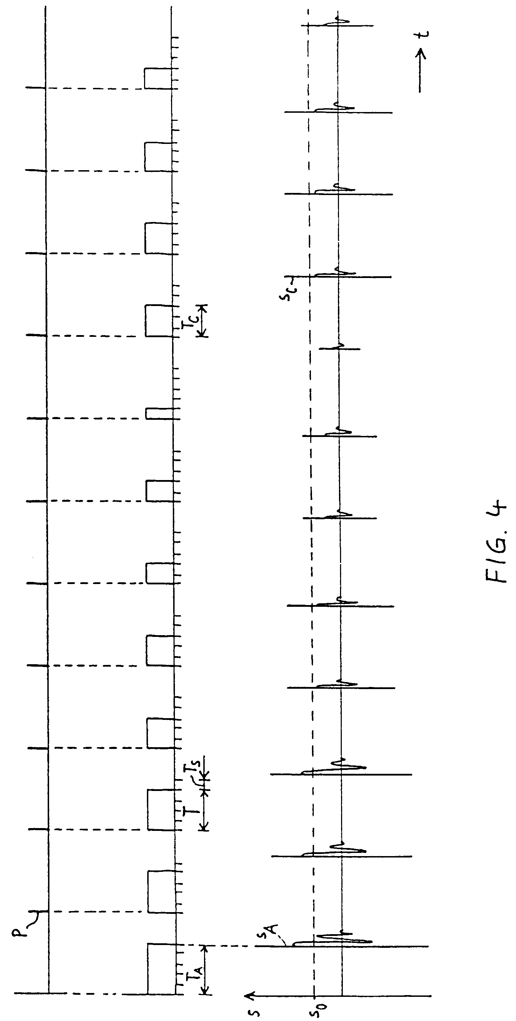

Entsprechend einer weiteren Ausführungsform der Erfindung, die im folgenden anhand von Figur 4 erläutert wird, ist das Einschaltzeitintervall T variabel. Mit den von dem Pumpratengeber 42 erzeugten Einzelimpulsen P ist jeweils der Beginn einer Pumpaktion markiert. Ausgehend von einem Wert für das Einschaltzeitintervall T, bei dem ein die Empfindlichkeitsschwelle $s_0$ mit Sicherheit übersteigendes Ausgangssignal s des Geräuschsensors 17 erhalten wird, also beispielsweise dem Wert $T_A$, wird das Zeitintervall T nach einer vorgegebenen Anzahl von Pumpaktionen (hier zwei) um eine vorgegebene Schrittweite $T_s$ verkürzt. Dieser Vorgang wird so oft wiederholt, bis das Ausgangssignal s des Geräuschsensors 17 die Empfindlichkeitsschwelle $s_0$ unterschreitet. Daraufhin wird das Zeitintervall um mindestens eine Schrittweite $T_s$ auf den Wert $T_c$ verlängert (FIG 3) und der neue Wert eine vorgegebene Zeit oder Anzahl von Pumpaktionen lang beibehalten, bis erneut durch schrittweises Verkürzen des Einschaltzeitintervalles T ein neuer Wert für das Einschaltzeitintervall ermittelt wird. Dieser Vorgang wird auch dann eingeleitet, wenn das Ausgangsignal des Geräuschsensors 17 plötzlich ausbleiben sollte. Aufgrund von Veränderungen in der Dosierpumpe 1, in dem Kathetersystem oder in externen Verhältnissen, wie Druck und Temperatur, oder in der zu fördernden Flüssigkeit kann sich nämlich die Kolbenbewegung verändern, so daß auch bei unverändertem Einschaltzeitintervall T für die elektromagnetische Erregung unterschiedliche Ausgangssignale der Detektoreinrichtung 17 erhalten werden. Durch das schrittweise Verkürzen des Einschaltzeitintervalls bis zum Ausbleiben des Anschlagens des Kolbens 4 gegen den Anschlag 29 und das nachfolgende Verlängern des Einschaltzeitintervalls um mindestens eine Schrittweite wird die Ansteuerung der Kolbenpumpe 4 automatisch an veränderte Pumpbedingungen angepaßt. Überschreitet dabei die Länge des Einschaltzeitintervalls einen vorgegebenen Maximalwert oder unterschreitet einen vorgegebenen Mindestwert, so wird über den Fehlermelder 49 eine Fehlermeldung abgegeben.

Durch das Abspeichern ausbleibender Detektionen des Kolbenanschlags zusammen mit der Pumprate werden Informationen erhalten, aufgrund derer Ursachen für Störungen im Pumpbetrieb ermittelt oder künftige Störungen im voraus detektiert werden können.

Das Auftreten des Kolbens 4 gegen den Anschlag 29 kann außer durch einen Geräuschsensor 17 auch durch andere Sensoren, beispielsweise optische Sensoren oder Näherungsschalter, detektiert werden.

## Patentansprüche

1. Dosiergerät zur gesteuerten Abgabe einer Flüssigkeit aus einem Vorratsbehälter mit einer Kolbenpumpe (1), deren Kolben (4) durch eine ein- und ausschaltbare elektromagnetische Betätigungseinrichtung (6 bis 10) aus einer Ruhelage gegen einen Anschlag (29) eines den Kolben (4) umgebenden Zylindergehäuses (5) bewegbar ist, mit einem Pumpratengeber (42), der für jede einzelne Pumpaktion ein Einschaltsignal für die Betätigungseinrichtung (6 bis 10) erzeugt, und mit einer Detektoreinrichtung (17), die beim Auftreffen des Kolbens (4) gegen den Anschlag (29) ein Ausgangssignal (s) erzeugt, das in einer Auswerteeinrichtung (37) zur Erzeugung eines Ausschaltsignals für die Betätigungseinrichtung (6 bis 10) herangezogen wird, **dadurch gekennzeichnet**, daß die Auswerteeinrichtung (37) das Ausschaltsignal nach Ablauf eines auf das Auftreten des Einschaltsignals (P) folgenden Einschaltzeitintervalls ($T_B$, $T_C$) erzeugt, dessen Länge kürzer bemessen ist, als die bei fehlerfreiem Betrieb des Dosiergeräts für die Bewegung des Kolbens (4) aus der Ruhelage gegen den Anschlag (29) benötigte Zeit, und daß das Einschaltzeitintervall ($T_B, T_C$) verlängert wird, wenn bei einer vorgegebenen Anzahl zurückliegender Pumpaktionen die Zahl der detektierten Anschläge des Kolbens (4) einen vorgegebenen Wert unterschreitet.

2. Dosiergerät nach Anspruch 1, **dadurch gekennzeichnet**, daß das Einschaltzeitintervall ($T_B$) ausreichend lang bemessen ist, um den Kolben (4) bei störungsfreiem Betrieb des Dosiergeräts gegen den Anschlag (29) zu beschleunigen, und daß die Auswerteeinrichtung (37) zur Verlängerung des Einschaltzeitintervalls ($T_B$) das Ausgangssignal (s) der Detektoreinrichtung (17) als Ausschaltsignal heranzieht.

3. Dosiergerät nach Anspruch 1, **dadurch gekennzeichnet**, daß das Einschaltzeitintervall ($T_B$) bei jedem Ausbleiben einer Detektion des Auftreffens des Kolbens (4) gegen den Anschlag (29) um einen vorgegebenen Betrag verlängert wird.

4. Dosiergerät nach Anspruch 1 oder 3, **dadurch gekennzeichnet**, daß die Auswerteeinrichtung (37) zur Ermittlung des für eine vollständige Pumpaktion kürzest möglichen Einschaltzeitintervalls ($T_C$) das Einschaltzeitintervall ausgehend von einem Wert ($T_A$, $T_B$), bei dem ein Ausgangssignal ($s_A$, $s_B$) der Detektoreinrichtung (17) erhalten wird, in aufeinanderfolgenden Pumpaktionen schrittweise verkürzt, bis das Ausgangsignal (s) der Detektoreinrichtung (17) ausbleibt, und daß anschließend das Einschaltzeitintervall (T) um einen vorgegebenen Betrag verlängert wird, wobei dieser Vorgang mit gleichmässigen Intervallen wiederholt wird um das Einschaltzeitintervall dynamisch an veränderten Energiebedarf der Pumpe anzupassen.

5. Dosiergerät nach Anspruch 4, **dadurch gekennzeichnet**, daß die Detektoreinrichtung (17) das Auftreffen des Kolbens (4) gegen den Anschlag (29) mit einer vorgegebenen Empfindlichkeitsschwelle ($s_0$) detektiert und daß die Schrittweite ($T_S$), mit der die Verkürzung des Einschaltzeitintervalls (T) erfolgt, auf einen Wert bemessen ist, der kleiner ist, als die Differenz zwischen dem Einschaltzeitintervall ($T_C$), bei dem der Kolben gerade noch den Anschlag (29) erreicht und dem Einschaltzeitintervall, bei dem das Auftreffen des Kolbens (4) gegen den Anschlag (29) durch Überschreiten der Empfindlichkeitsschwelle ($s_0$) detektiert wird.

6. Dosiergerät nach einem der vorangehenden Ansprüche, **dadurch gekennzeichnet**, daß an der Auswerteeinrichtung (37) ein Fehletmelder (49) angeschlossen ist, der ein Fehlermeldungssignal abgibt, wenn die eingestellte Länge des Einschaltzeitintervalls (T) einen vorgegebenen Maximalwert überschreitet oder einen vorgegebenen Mindestwert unterschreitet.

7. Dosiergerät nach einem der Ansprüche 1 bis 5, **dadurch gekennzeichnet**, daß die Auswerteeinrichtung (37) ein eventuelles Fehlermeldungssignal bei Kommunikation zwischen der Telemetrieeinrichtung (39) und dem äußeren Programmiergerät (40) oder bei Anfrage von dem Arzt abgibt.

8. Dosiergerät nach einem der vorangehenden Ansprüche, **dadurch gekennzeichnet**, daß an der Auswerteeinrichtung (37) ein Datenspeicher (38) angeschlossen ist, in den beim Ausbleiben eines Ausgangssignals (s) der Detektoreinrichtung (17) der aktuelle Wert für das Einschaltzeitintervall (T) eingelesen wird.

9. Dosiergerät nach einem der Ansprüche 1 bis 7, **dadurch gekennzeichnet**, daß an der Auswerteeinrichtung (37) ein Datenspeicher (38) angeschlossen ist, in dem der aktuelle Wert für das Einschaltzeitintervall (T) fortlaufend eingelesen wird.

10. Dosiergerät nach Anspruch 7, **dadurch gekennzeichnet**, daß zusammen mit dem Wert für das Einschaltzeitintervall (T) der zugehörige aktuelle Wert der Pumprate abgespeichert wird.

11. Dosiergerät nach Ansprüchen 8 oder 9 und 10, **dadurch gekennzeichnet**, daß der zugehörige aktuelle Wert des Anschlagszeitintervalls abgespeichert wird.

12. Dosiergerät nach Ansprüchen 8 oder 9 und 10 bis 11, **dadurch gekennzeichnet**, daß der zugehörige aktuelle Wert des Zeitpunkts ($t_1$) des Beginns von der Beschleunigung des Kolbens (4) abgespeichert wird.

13. Dosiergerät nach einem der Ansprüche 8 oder 9 und den Ansprüchen 10 bis 12, **dadurch gekennzeichnet**, daß die Auswerteeinrichtung (37) die abgespeicherten Pumpenparameter auswertet und daraus festlegt, ob eine eventuelle Fehlermeldung über den Fehlermelder (49), bei Kommunikation zwischen der Telemetrieeinrichtung (39) und dem äußeren Programmiergerät (40) oder bei Anfrage vom Arzt abgegen werden soll.

**Claims**

1. Dosing device for the controlled release of a liquid from a reservoir, having a piston pump (1) whose piston (4) can be moved by an electromagnetic actuating device (6 to 10), which can be turned on and off, from a quiescent position to a point against a stop (29) of a cylinder housing (5) surrounding the piston (4), having a pump rate transmitter (42) which generates a turn-on signal for the actuating device (6 to 10) for each individual pump action, and having a detector device (17) which generates an output signal (s) when the piston (4) strikes against the stop (29), which output signal is used in an evaluation device (37) for generating a turn-off signal for the actuating device (6 to 10), characterized in that the evaluation device (37) generates the turn-off signal after the expiry of an on-time interval ($T_B$, $T_C$) which follows the occurrence of the turn-on signal (P) and whose length is made to be shorter than the time required for the movement of the piston (4) from the quiescent position to a point against the stop (29) given fault-free operation of the dosing device, and in that the on-time interval ($T_B$, $T_C$) is lengthened when, given a predetermined number of preceding pump actions, the number of detected strikes of the piston (4) falls below a predetermined value.

2. Dosing device according to Claim 1, characterized in that the on-time interval ($T_B$) is made to be long enough to accelerate the piston (4) against the stop (29) given fault-free operation of the dosing device, and in that the evaluation device (7), for the purpose of lengthening the on-time interval ($T_B$), uses the output signal (s) of the detector device (17) as turn-off signal.

3. Dosing device according to Claim 1, characterized in that the on-time interval ($T_B$) is lengthened by a predetermined amount each time a detection of the striking of the piston (4) against the stop (29) fails to appear.

4. Dosing device according to Claim 1 or 3, characterized in that the evaluation device (37), for the purpose of determining the shortest possible on-time interval ($T_C$) for a complete pump action, shortens step by step the on-time interval, starting from a value ($T_A$, $T_B$) at which an output signal ($s_A$, $s_B$) of the detector arrangement (17) is received, in successive pump actions until the output signal (s) of the detector device (17) fails to appear, and in that the on-time interval (T) is subsequently lengthened by a predetermined amount, this procedure being repeated at uniform intervals for the purpose of dynamically adapting the on-time interval to an altered energy requirement of the pump.

5. Dosing device according to Claim 4, characterized in that the detector device (17) detects the striking of the piston (4) against the stop (29) with a predetermined sensitivity threshold ($s_0$), and in that the step width ($T_s$) by which the on-time interval (T) is shortened is given a value which is less than the difference between the on-time interval ($T_c$) at which the piston still just reaches the stop (29) and the on-time interval at which the striking of the piston (4) against the stop (29) is detected by the upwards transgression of the sensitivity threshold ($s_0$).

6. Dosing device according to one of the preceding claims, characterized in that an error detector (49) is connected to the evaluation device (37) and emits an error message signal when the set length of the on-time interval (T) exceeds a predetermined maximum value or falls below a predetermined minimum value.

7. Dosing device according to one of Claims 1 to 5, characterized in that the evaluation device (37) emits a possible error message signal in the event of communication between the telemetry device (39) and the external programming device (40) or in the event of an enquiry from the doctor.

8. Dosing device according to one of the preceding claims, characterized in that a data memory (38) is connected to the evaluation device (37) and the current value for the on-time interval (T) is read into the said data memory when an output signal s of the detector device (17) fails to appear.

9. Dosing device according to one of Claims 1 to 7, characterized in that a data memory (38) is connected to the evaluation device (37) and the current value for the on-time interval (T) is continuously read into the said data memory.

10. Dosing device according to Claim 7, characterized in that the associated current value of the pump rate is stored together with the value for the on-time interval (T).

11. Dosing device according to Claims 8 or 9 and 10, characterized in that the associated current value of the striking time interval is stored.

12. Dosing device according to Claims 8 or 9 and 10 to 11, characterized in that the associated current value of the instant ($t_1$) of the beginning of the acceleration of the piston (4) is stored.

13. Dosing device according to either of Claims 8 and 9 and Claims 10 to 12, characterized in that the evaluation device (37) evaluates the stored pump parameters and determines therefrom whether a possible error message is to be emitted via the error detector (49), in the event of communication between the telemetry device (39) and the external programming device (40) or in the event of an enquiry by the doctor.

**Revendications**

1. Appareil de dosage pour la fourniture commandée d'un liquide provenant d'un réservoir, comportant une pompe (1) à piston dont le piston (4) peut être déplacé par un dispositif (6 à 10) d'actionnement électromagnétique pouvant être branché et débranché d'une position de repos à une butée (29) d'un corps (5) de cylindre entourant la piston (4), comportant un indicateur (42) de vitesse de pompage qui produit à chaque actionnement individuel de la pompe un signal de branchement destiné au dispositif (6 à 10) d'actionnement, et comportant un dispositif (17) à détecteur qui, lors de l'arrivée du piston (4) à la butée (29), produit un signal (s) de sortie qui est utilisé dans un dispositif (37) d'exploitation pour produire un signal de débranchement destiné au dispositif (6 à 10) d'actionnement, caractérisé en ce que la dispositif (37) d'exploitation produit le signal de débranchement après que s'est écoulé un intervalle ($T_B$, $T_C$) de temps de branchement qui succède à l'arrivée du signal (P) de branchement et dont la durée est choisie plus brève que la durée qui est nécessaire lors du fonctionnement sans incident de l'appareil de dosage pour la déplacement du piston (4) de la position de repos à la butée (29), et en ce que l'intervalle ($T_B$, $T_C$) de temps de débranchement est prolongé si, pour un nombre prescrit d'actionnement de la pompe précédents, le nombre des butées détectées du piston (4) est inférieur à une valeur prescrite.

2. Appareil de dosage suivant la revendication 1, caractérisé en ce que i'intervalle ($T_B$) de temps de branchement est choisi suffisamment long pour accélérer le piston (4) vers la butée (5) en fontionnement sans incident de l'appareil de dosage et en ce que le dispositif (37) d'exploitation utilise la signal (s) de sortie du dispositif (17) à détecteur comme signal de débranchement pour prolonger l'intervalle ($T_B$) de temps de branchement.

3. Appareil de dosage suivant la revendiction 1, caractérisé en ce que l'intervalle ($T_B$) de temps de branchement est prolongé d'une durée prescrite chaque fois qu'une détection de l'arrivée du piston (4) à la butée (29) fait défaut.

4. Appareil de dosage suivant la revendication 1 ou 3, caractérisé en ce que, pour déterminer l'intervalle ($T_C$) de temps de branchement possible le plus court pour un actionnement complet de la pompe, le dispositif (37) d'exploitation écourte pas à pas, au cours d'actionnement successifs de la pompe, l'intervalle ($T_C$) de temps de branchement, à partir d'une valeur ($T_A$, $T_B$) pour laquelle un signal ($s_A$, $s_B$) de sortie du dispositif (17) à détecteur est obtenu, jusqu'à ce que le signal (s) de sortie du dispositif (17) à détecteur fasse défaut, et en ce que, ensuite, l'intervalle (T) de temps de branchement est prolongé d'une durée prescrite, cette opération étant répétée à des intervalles réguliers pour adapter l'intervalle de temps de branchement de manière dynamique à des besoins en énergie modifiés de la pompe.

5. Appareil de dosage suivant la revendication 4, caractérisé en ce que le dispositif (17) à détecteur détecte l'arrivée du piston (4) à la butée (29) par un seuil ($s_O$) prescrit de sensibilité et en ce que la largeur de pas ($T_S$) avec laquelle la diminution de i'intervalle (T) de temps de branchement s'effectue est établie à une valeur qui est inférieure à la différence entre l'intervalle ($T_C$) de temps de branchement pour lequel le piston atteint encore juste la butée (29) et l'intervalle de temps de branchement pour lequel l'arrivée du piston (4) à la butée (29) est détectée par le dépassement du seuil ($s_O$) de sensibilité.

6. Appareil de dosage suivant l'une des revendication précédentes, caractérise en ce qu'il est raccordé au dispositif (37) d'exploitation un dispositif (49) de signal d'erreur qui fournit un signal d'avertissement d'erreur si la longueur réglée de l'intervalle (T) de temps de branchement est supérieure à une valeur maximum prescrite ou est inférieure à une valeur minimum prescrite.

7. Appareil de dosage suivant l'une des revendications 1 à 5, caractérisé an ce que la dispositif (37) d'exploitation fournit un éventuel signal d'avertissement d'erreur lors d'une communication entre le dispositif (39) de télémétrie et l'appareil (40) de programmation extérieur, ou à la demande du médecin.

8. Appareil de dosage suivent l'une des revendications précédentes, caractérisé en ce qu'il est raccordé au dispositif (37) d'exploitation un dispositif (38) de mémorisation de données, dans lequel la valeur instantanée de l'intervalle (T) de temps de branchement est lue lorsqu'un signal (s) de sortie du dispositif (17) à détecteur fait défaut.

9. Appareil de dosage suivant l'une des revendications 1 à 7, caractérisé en ce qu'il est raccordé au dispositif (37) d'exploitation un dispositif (38) de mémorisation de données, dans lequel la valeur instantanée de l'intervalle (T) de temps de branchement est lue en permanence.

10. Appareil de dosage suivant la revendications 7, caractérisé en ce que la valeur instantanée associée de la vitesse de pompage est mémorisée conjointement avec la valeur affectée à l'intervalle (T) de temps de branchement.

11. Appareil de dosage suivant les revendications 8 au 9 et 10, caractérisé an ce que la valeur instantanée associée de l'intervalle de temps de butée est mémorisée.

12. Appareil de dosage suivant les revendications 8 ou 9 et 10 à 11, caractérisé en ce que la valeur instantanée associée de l'instant ($t_1$) du début de l'accélération du piston (4) est mémorisée.

13. Appareil de dosage suivant l'une des revendications 8 ou 9 et les revendications 10 à 12, caractérisé en ce que le dispositif (37) d'exploitation exploite les paramètres de la pompe mis en mémoire et en détermine si un éventuel message d'erreur doit être fournl par l'intermédiaire de dispositif (49) de signal d'erreur, lors d'une communication entre la dispositif (39) de télémétrie et l'appareil (40) de programmation extérieur, ou à la demande du médecin.

FIG 1

FIG 2

FIG. 3

FIG. 4